# EUROPEAN PATENT APPLICATION

(11) **EP 3 251 688 A1**
(43) Date of publication of application: **06.12.2017**
(21) Application number: 16715343.6
(22) Date of filing: 28.01.2016
(51) Int. Cl.: A61K 38/14, A61K 47/26, A61K 9/10, A61K 31/337, A61K 31/7048, A61P 35/00

(54) **WATER-SOLUBLE PHARMACEUTICAL COMPOSITION COMPRISING AT LEAST ONE THERAPEUTICALLY ACTIVE SUBSTANCE AND AT LEAST ONE SUBSTANCE CAPABLE OF FORMING MICELLES**

(30) Priority: 30.01.2015 AR P150100269
(71) Applicant: Consejo Nacional de Investigaciones Científicas Y Técnicas (CONICET), Buenos Aires C1425FQB (AR); Centro de Excelencia en Productos y Procesos Córdoba, Córdoba - Prov. de Córdoba, X5004AAP (AR); Beltramo, Dante Miguel, Córdoba - Prov. de Córdoba X5010 (AR)
(72) Inventor: BELTRAMO, Dante Miguel, Córdoba - Prov. de Córdoba X5010 (AR); ALASINO, Roxana Valeria, Córdoba - Prov. de Córdoba X5004AAP (AR); GARCIA, Néstor, Córdoba - Prov. de Córdoba X5004AAP (AR)
(74) Representative: Lang, Johannes
(86) International application number: PCT/IB2016/050437
(87) International publication number: WO 2016/120824

(57) **Abstract**

A water-soluble pharmaceutical composition comprising at least a therapeutically active substance and at least a substance capable of forming micelles, wherein such therapeutically active substance is selected from drugs with hydrophobic characteristics to treat cancer patients and wherein such substance capable of forming micelles is selected from lipoglycopeptide antibiotics, such as Teicoplanin, Dalbavancin and Oritavancin.

## Description

This invention is a water-soluble pharmaceutical composition comprising, at least, a therapeutically active substance and, at least, a substance capable of forming micelles, wherein such therapeutically active substance is selected from drugs with hydrophobic characteristics for the treatment of cancer patients and wherein such substance capable of forming micelles is selected from Teicoplanin, Dalbavancin and Oritavancin compounds.

This way, this invention refers to a water-soluble pharmaceutical composition comprising, as therapeutically active substances, one or more hydrophobic drugs used for the treatment of cancer patients and also one or more compounds with lipoglycopeptide structure, which have bactericidal properties and the ability to self-assemble into micelles which allow the solubility and transportation of such hydrophobic drugs. In particular, this invention refers to a sterile and injectable composition, made by micelles from antibiotics known as lipoglycopeptides, which spontaneously and non-covalently interact with albumin and allow the solubility, transportation and release of hydrophobic molecules used for tumor treatment.

### BACKGROUND OF THE INVENTION

The effectiveness of many drugs, especially of those with hydrophobic characteristics, is limited mainly by the inability to reach the correct site of therapeutic action. In many cases, and even when the drug is water-soluble, only a small fraction of the administered dose reaches the therapeutic site, while most of the drug is distributed throughout the body. This way, the distribution in healthy organs and tissues often limits the dose. Therefore, a pharmaceutical formula containing strong and ideal hydrophobic active ingredients would be one that allows high solubility of the drug in aqueous medium, stable as no-solution complex, staying as long as possible in blood, increasing the possibility of reaching tumor cells and that is, at the same time and if possible, only on the specific site of action.

Among the active substances showing hydrophobic nature and having very low or limited water solubility are, for example, those used in cancer treatment (such as Paclitaxel and Docetaxel) and within the antifungal ones, Amphotericin B.

Paclitaxel (Ptx) is a molecule that interacts and promotes tubulin polymerization to form highly stable microtubules (MT). These results of MT stabilization produce the inhibition of the normal dynamics of reorganization of the microtubule network. This does not happen with other anti-microtubule agents such as, for example, colchicine, vincristine or vinblastine, which produce the disassembly of MT network.

One of the main problems in the development of drugs that have Ptx is the high water insolubility. The formulations of drugs with low water solubility have traditionally been developed in the shape of emulsions or by combining actives with colloids, such as micelles. Colloids can dissolve the active, thus increasing its concentration in aqueous medium.

In particular, Ptx has very low water solubility (about 1 *µ*g/ml). Therefore, several pharmaceutical companies and authors have proposed different approaches for the development of Ptx formulations for intravenous infusion. This way, it has been shown that some organic solvents are capable of partially dissolving Ptx. However, when a miscible solvent is diluted in aqueous medium with the water containing Ptx and in which the latter has a concentration close to its saturation point, the drug begins to precipitate.

The injectable form of Ptx is currently internationally marketed by Bristol-Myers Squibb Co. (New York, NY) in a single dose of 30 mg (5 ml).

Each milliliter of the formulation contains approximately 6 mg of Ptx, 527 mg of Cremophor EL, and 49.7% (v/v) of dehydrated alcohol. For administration, this concentrated formulation may be diluted with saline solution, 5% dextrose in water, 5% dextrose in saline or Ringer solution with 5% dextrose in water (Goldspiel, 1994, "Pharmaceutical Issues: Preparation, Administration, Stability, and Compatibility with Other Medications "Ann. Pharmacotherapy, vol. 28, pp. S23-26. Harvey Whitney Books Company). However, it should be noted that the formulation of Ptx in Cremophor/ethanol when diluted in aqueous medium, it begins to become unstable, showing over time fibrous precipitates. Ptx formulations in Cremophor are disclosed in US patent 5504102.

As with most chemotherapy agents, the maximum tolerated dose of Ptx is limited by its toxicity. In humans, the biggest toxic effects of Ptx are observed at concentrations ranging from about 100 to 250 mg/m². For example, among the adverse effects mentioned in books, there are granulocytopenia (Holmes F. A.et al., "Phase II trial of taxol, an active drug in the treatment of metastatic breast cancer", J Natl Cancer Inst. 1991, 83:1797-1805) and symptomatic peripheral neuropathy, being the latter the main non-haematological toxicity (Rowinsky E. K. et al, 1995, Review Article. Drug Therapy - Paclitaxel (Taxol) N. Engl. J. Med. Vol 332:1004-1014 N.15).

Recently, Ptx was prepared in a formulation both in lyophilized solid state and in liquid state for its use in intravenous form. In US patent 6743826, a method for obtaining soluble Ptx through interaction with human albumin or recombinant human albumin is described. Then, in US patent application 2005282734 A1, a lyophilized formulation or a water-soluble composition containing Ptx or Docetaxel attached to human albumin, wherein the concentration of Ptx is higher than 500 ug/ml is described. However, in such application, it is not explicitly stated which would be the solubility of Ptx in the formulation.

The albumin molecule has a key role in the transportation of hydrophobic molecules, such as Ptx. Its direction in different tissues is related to the ability of interaction of albumin with cell receptors and cell accumulation (Gradishar W. J., 2006, "Albumin-bound paclitaxel: A next-generation taxane, Expert Opin Pharmacother; 7:1041-1053). The albumin-Ptx complex is reversible, a condition that allows it to be carried in the body and to be released in the cell surface. Through the link to the receptor, albumin starts the so-called transcytosis of albumin-Ptx complex through the wall of endothelial cells of blood vessels, thus making easier the passage of albumin-Ptx complex (called ABI-007) to the interstitial complex and, therefore, allowing direct exposure to tumor surface (Ibrahim N. K. et al., 2002, "Phase I and pharmacokinetic study of ABI-007, a Cremophor-free, protein-stabilized, nanoparticle formulation of paclitaxel," Clin Cancer Res-;8:1038-1044; Gradishar W. J., 2006, "Albumin-bound paclitaxel: A next-generation taxane," Expert Opin Pharmacother, 7:1041-1053; Drummond D. C. et al., 1999, "Optimizing liposomes for delivery of chemotherapeutic agents to solid tumors." Pharmacol Rev.; 51:691-743). Albumin accumulates in tumors, possibly and partly, as a result of the secretion of the so-called albumin linking protein (SPARC, secreted protein, acidic and rich in cysteine, also called BM-40), which can result in intratumoral accumulation of Ptx linked to albumin (Fukunaga-Kalabis M. and Herlyn M., 2007, "Unraveling mysteries of the multifunctional protein SPARC," J Invest Dermatol;127: 2497-2498). Moreover, recently, other strategies to obtain water-soluble Ptx have been disclosed in specialized books, such as, for example, by it conjugation to polymers such as polyglutamic ones (US Patent 7384977).

The action mechanisms of anticancer or encapsulated antifungal drugs are not fully understood, but it is believed that they could result or be consequence of the ability of liposomes to release slowly into the bloodstream an encapsulated drug, or from the interaction of liposome with the surface of the tumor to slowly release the drug directly into the tumor site. However, a serious problem of encapsulated drugs in liposomes is that it has been found that these drugs are rapidly released from liposomes after encapsulation. Moreover, highly lipophilic drugs (water-insoluble) that partition in the lipid bi-layer of a liposome seem, in some cases, to strongly modify the physical properties of the membranes to such an extent that the membrane itself becomes unstable and it cannot hold the drug releasing it into to the environment. Based on these remarks, it should be achieved that in an encapsulated drug system, such as liposomes, the liposome can be protected stabilizing it in order to prevent these unwanted liposome-drug interactions.

A very important property that should be achieved in these formulations is an increase in relation to plasma circulation time. Biodistribution tests are performed on specifically selected organs or tissues, analyzing the contents of lipids of the liposomes used or the drugs under analysis, by samples of tissue homogenization. Adding to limposomes lipid molecules with sugars or lipids containing amino groups (or carboxyl, in the case of monosialogangliosides), such as monosialoganglioside GM1, significantly reduces the accumulation of the lipids of liposome in the liver and spleen. For example, in the case of vincristine drug, a formulation combines an internal pH of the liposome of 2 and the presence of GM1 in the liposome bi-layer. Intravenous administration of this formulation containing 2, 3 or 4 mg/kg of drug to mice inoculated with tumor cells P388 was able to produce a great survival in animals compared to the controls. Several laboratories have also assessed the possibility of increasing the half-life of circulation of liposomes designing artifices for their surface, making them similar to red blood cells (RBCs).

The role of surface carbohydrates on cell recognition is a widely analyzed phenomenon (Ashwell G. and Morell A. G., 1974, "The role of surfacecarbohydrates in the hepatic recognition and transport of circulating glycoproteins", Adv Enzymol 41:99-128; Hakormori S., 1981, "Glycosphingolipids in cellular interaction, differentiation, and oncogenesis." Annu Rev Biochem. Vol. 50:733-764). In addition, the chemistry, metabolism and biological functions of gangliosides and sialic acid have been deeply analyzed (Schauer, R., 1982, "Chemistry, metabolism, and biological functions of sialic acids", Adv. Carbohydrate Chem. Biochem. 40, 131-23; Ledeen R. W. et al., 1998, "Sphinglolipids as signaling modulators in the nervous system", Annals of the New Cork Academy of Science, Vol 845). It has been reported that the incorporation of ganglioside GM1 into liposomes made up of phosphatidylcholine (PC) and cholesterol significantly increases the half-life of their circulation (Bedu-Addo F. K. and L. Huang, 1996, "Effect of matrix lipid chain length on liposomes containing cholesterol and ganglioside GM1: Implications in Drug Delivery ", Journal of Pharmaceutical Sciences, Volume 85, Issue 7, Pages 714-719).

The above results and comments clearly show the current need to develop formulations containing hydrophobic, water-soluble substances and able to contain effective amounts of such active substances such as, for example, Paclitaxel and Docetaxel, but not showing the disadvantages caused by the insolubility of the above drugs.

Other strategies for the formulation of drugs poorly water-soluble has traditionally been the use of emulsions and other colloidal associations, such as, for example, micelles, a type of structure that dissolves the drug and also increases its concentration in aqueous medium (Patent 6296870).

A micelle is a colloidal association that has regions having a strong anisotropy and a gradient of water solubility that decreases from out to inward. Therefore, micelles are capable of turning soluble a wide range of solutes.

Micelles can turn soluble insoluble organic matter due to its ability to incorporate such matter in their highly hydrophobic region. In the outermost part of the hydrocarbon chain, the three or four carbon atoms are all trans and, therefore, is a less fluent domain. Therefore, this region, as it is weakly hydrophobic, may be partially hydrated. Thus, a transition area is achieved between the purely hydrophobic areas and the purely hydrophilic ones. Molecules entering this area should be slightly compatible both with the hydrophobic area of the lipid chain and the solvated area of the polar head region and, therefore, they must behave as amphipathic molecules. In the case of active ingredients with hydrophobic characteristics, they tend to fix directly into the deep region of the micelle through the interaction with hydrophobic chains of fatty acids. Regarding the polar head of micelles, it is possible to achieve a wide range of compositions. This implies the possibility of obtaining micelles with surfaces having different characteristics and that could be used to associate different active substances. In the case of ionic micelles, the region of polar head can be fixed to a large number of conjugated ions. Therefore, it is similar to a concentrated electrolyte solution. Moreover, the diffuse ionic bi-layer in the charged micelles extends over the so called Stern layer.

Micelles can dissolve active agents and therefore they are an excellent system to capture and incorporate insoluble or partially insoluble molecules. However, it has been stated that the use of micelles in these systems could be limited to relatively small molecules that can adapt to the highly anisotropic structure of the fatty acid chains. This could be by virtue of occupying the intermediate region or due to the fact that they can adapt within the fatty acid chains.

Emulsions and, especially, micelle suspensions are not necessarily stable and they may not reach to places of interest. However, it is known that emulsions and micelles loaded with active agents have higher motility than liposomes. Generally, micelles are considered stable only when in balance with the surfactant monomer that makes them up. Therefore, in the absence of balance monomer, micelles can break apart in the water-soluble monomer to finally completely dilute. Similarly, in the absence of balance monomer, micro-emulsion droplets may coalesce to form larger droplets which eventually become lost. Therefore, when a micelle preparation is diluted, for example due to intravenous administration, they can be dissolved and dispersed in the medium, with leaks of their contents into the bloodstream in seconds. Similarly, preparations in the shape of emulsion show the same principle of instability.

Recently, it was published the patent application US20130195924A1 "Water-soluble pharmaceutical composition comprising at Least one therapeutic active substance having hydrophobic properties and at least one compound selected from a monosialoglycosphinglolipids, glycosphingolipids or a mixture of sialoglycosphingolipids and glycosphingolipids." It, and in order to solve some of the above mentioned problems, proposes that the micelles of GM1 monosialogangliosides are capable of making soluble or fixing in a stable manner hydrophobic drugs, such as Paclitaxel or Amphotericin B. This property is due, one the one hand, to the low critical micellar concentration (cmc) of GM1, which is in the order of 10⁻⁸ 10⁻¹⁰ M and, on the other hand, to the fact that the presence of the hydrophobic drug within GM1 micelles produces an increase in the stability of micelles, as shown by dialysis experiments. Thus, GM1 decreases by 32% after 72 hours of dialysis, while the GM1-Ptx complex decreases by only 3%.

Moreover, the use of lipopeptide micelles as drug transporting agents is described in the European patent application EP2264047(A1) "High purity lipopeptides, lipopeptide micelles and processes for preparing same." In such document, it is essentially described the purification and characterization of the physicochemical properties of lipopeptides, their ability to form micelles, as well as a complete characterization of their properties as antibiotics. In particular, it is disclosed the use of a particular cyclic lipopeptide known as Daptomycin (Dapto), which is active against Gram positive bacteria. Generally, in such publication, it is mentioned the potential use of antibiotic lipopeptides as hydrophobic drugs carriers. In such document, it is not described the use of lipoglycopeptide micelles for the specific incorporation of drugs with hydrophobic characteristics for the treatment of cancer patients.

### BRIEF DESCRIPTION OF THE INVENTION

This invention refers to a water-soluble pharmaceutical composition comprising, at least, a therapeutically active substance and, at least, a substance capable of forming micelles, wherein such therapeutically active substance is selected from drugs with hydrophobic characteristics for the treatment of cancer patients and wherein such substance capable of forming micelles is selected from Teicoplanin, Dalbavancin and Oritavancin compounds.

### DESCRIPTION OF THE FIGURES

Figure 1. Chromatographic profile in Sephadex G200 from the Teicoplanin-Paclitaxel complex -○-, and of the Teicoplani-Paclitaxel-Albumin complex -□-.
Figure 2. Graph determining particle size of Teicoplanin by the technique of dynamic dispersion of light. At the right bottom, there is the information of particle size in nanometers.
Figure 3. Graph determining particle size of Teicoplanin-Paclitaxel complex by the technique of dynamic dispersion of light. At the right bottom, there is the information of particle size in nanometers.
Figure 4. Graph determining particle size of Teicoplanin-Paclitaxel-Albumin complex by the technique of dynamic dispersion of light. At the right bottom, there is the information of particle size in nanometers.
Figure 5. Electron micrograph at 150,000 x for Teicoplanin complex particles. The black bar represents 200 nm.
Figure 6. Electron micrograph at 250,000 x for Teicoplanin-Paclitaxel complex particles. The green numbers represent the size of micelles.
Figure 7. Electron micrograph at 250,000 x for Teicoplanin-Paclitaxel-Ablumin complex particles. The green numbers represent the size of micelles.

### DETAILED DESCRIPTION OF THE INVENTION

In order to properly use a nanostructure of micelles as a system of drug loading, transportation and delivery into the bloodstream, it is necessary to improve the behavioral problems of nanostructures of the former art, according to the following aspects:
1 - avoiding problems related to the dissolution of micelles caused by the phenomenon of dilution,
2 - obtaining a structure with a surface or with electrical and/or physicochemical properties that prevent the interaction with red blood cells,
3 - achieving that micelles are coated with polymers or proteins that shall: i) stabilize the complexes of micelles with the drugs and/or ii) act as agents so that micelles can reach the desired tissue.
4 - obtaining a structure significantly small (lower than 100 nm) to prevent the fast elimination from the bloodstream by the reticuloendothelial system.
5 - obtaining a structure with a molecular weight higher than 40 kDa, in order to prevent the fast elimination by the renal system.

The inventors of this invention have developed a new strategy that greatly improves the problems of the former art and, in particular, they have developed novel pharmaceutical compositions based on stable nanomicelles that allow loading high concentrations of therapeutically active substances in general and of drugs with hydrophobic characteristics for the treatment of cancer patients in particular.

Thus, this invention refers to a water-soluble pharmaceutical composition comprising, at least, a therapeutically active substance and, at least, a substance capable of forming micelles, wherein such therapeutically active substance is selected from drugs with hydrophobic characteristics for the treatment of cancer patients and wherein such substance capable of forming micelles is selected from Teicoplanin, Dalbavancin and Oritavancin compounds.

In a particular embodiment, the composition of the invention comprises drug with hydrophobic characteristics, useful in treating cancer patients, and that are selected from anticancer drugs. In preferred embodiments, the composition of the invention comprises at least a drug selected from Paclitaxel and Docetaxel. An expert in the art shall know other drugs with hydrophobic characteristics that could be incorporated into the compositions of this invention, thus obtaining the advantages described herein. For example, the compositions of this invention could incorporate hydrophobic antifungal drug, such as Amphotericin B.

It is a particular object of the invention a water-soluble pharmaceutical composition comprising, at least, an antitumor drug with hydrophobic characteristics and, at least, a substance capable of forming micelles selected from Teicoplanin, Dalbavancin and Oritavancin compounds, wherein such compounds are forming micelles and wherein such antitumor drug with hydrophobic characteristics is fixed to micelles non-covalently. In an especially preferred embodiment, the above composition comprises, at least, a drug selected from Paclitaxel and Docetaxel.

Another particular object of the invention is a water-soluble pharmaceutical composition comprising an antitumor drug with hydrophobic characteristics and a substance capable of forming micelles, wherein such antitumor drug is Paclitaxel and wherein such substance capable of forming micelles is Teicoplanin. In an especially preferred embodiment, the composition of the invention comprises Paclitaxel and Teicoplanin in a molar ratio of about 1:5 and 1:100. In another especially preferred embodiment, the water-soluble pharmaceutical composition of the invention comprises from about 1 mg/ml to about 10 mg/ml of Paclitaxel and from about 10 mg/ml to about 200 mg/ml of Teicoplanin.

Another particular object of the invention is a water-soluble pharmaceutical composition comprising an antitumor drug with hydrophobic characteristics and a substance capable of forming micelles, wherein such antitumor drug is Docetaxel and wherein such substance capable of forming micelles is Teicoplanin. In an especially preferred embodiment, the composition of the invention comprises Docetaxel and Teicoplanin in a molar ratio of about 1:5 and 1:100. In another especially preferred embodiment, the water-soluble pharmaceutical composition of the invention comprises from about 1 mg/ml to about 10 mg/ml of Docetaxel and from about 10 mg/ml to about 200 mg/ml of Teicoplanin.

In a particular embodiment of the invention, the soluble pharmaceutical composition is a sterile injectable composition. In an especially preferred embodiment, the water-soluble pharmaceutical composition of the invention is a lyophilized composition.

In particular embodiments of the invention, the composition is lyophilized and it can be reconstituted with a solvent selected from a solution of distilled water, a saline solution (NaCl 0.9%), a phosphate buffer saline (PBS) solution, distilled water containing 5% of dextrose, saline solution with 5% of dextrose and a human albumin solution at 20%, in order to obtain a sterile translucent composition.

According to this invention, "micelle" means a colloidal association having regions with a high anisotropy and a gradient of decreasing water solubility, going from the outside to the inside of it.

Among other advantages of the invention, we can mention the following ones:
1- Teicoplanin, Dalbavancin and Oritavancin micelles are capable of spontaneously incorporating therapeutically active substances with hydrophobic properties, useful for the treatment of cancer patients. In particular, they are able to incorporate spontaneously Paclitaxel and Docetaxel.
2- An outstanding property of a composition according to the invention comprising Teicoplanin forming micelles is that it shows the highest plasma circulation time possible. It is known that liposomes are structures that are quickly eliminated from blood (15 to 30 minutes), due to their interaction with cells of the reticuloendothelial system (RES). Therefore, such structures should be modified, for example, by adding lipids with sugars or with polymers such as, for example, polyethylene glycol (PEG). It has been described that adding PEG increases the circulation time on blood of liposomes, from 30 min. to about 40 hours. On the other hand, a composition according to the invention containing Teicoplanin may have a plasma circulation time much higher, being possible to reach a half-life of about 70 to 100 hours.
3. A composition according to the invention comprising Teicoplanin forming micelles may be spontaneously coated with albumin (Alb) (Bonati, M., G. L. Traina, R. Rosina and G. Buniva (1988) J. Antimicmb. Chemother 21 (Suppl. A):. 29-37) to offer a specific interaction of micelle with tumor. In this regard, it has been previously described that albumin, in addition to prevent removal of nanostructures by the reticuloendothelial system (RES), it is able to interact with cell receptors and to start the mechanism of transcytosis through the capillary endothelial cells. Through this mechanism, it would be possible the passage of this molecule of albumin and of related molecules, in order to place them near the surface of the tumor. According to particular embodiments of this invention, Teicoplanin micelles incorporating Paclitaxel (Ptx) or Docetaxel (Dtx) to make up the Teicoplanin-Paclitaxel (Teico-Ptx) complex or Teicoplanin-Docetaxel (Teico-DTX) complex have the ability to fix to albumin. Thus, it is possible to generate a ternary complex Alb-Teico-Ptx or Alb-Teico-Dtx, which can be used to reach the tumor cells.
4- Nanostructure size of micelles of compositions according to this invention is also an important factor, since molecules or complexes having a molecular weight (MW) less than 40 kDa are rapidly filtered by the kidney. According to this invention, the nanostructure of Teico-Ptx complex has a MW of about 50 to 60 kDa and Teico-Ptx-Alb complex has a MW of about 250 to 300 kDa.
5- Another important property of injectable formulations of anti-tumor drugs is that they avoid any interaction with red blood cells (RBCs), which represent the first cell hyper-surface with which they could interact, releasing their contents. According to this invention, neither Teicoplanin nor Teico-Ptx complex interact with red blood cells (RBCs), although they can rapidly reach to and interact with various tissues, such as the skin, synovial and peritoneal cavity fluids, the pericardium, the pleura, the bones, the kidney, the trachea, the adrenal glands, the fat tissue, the cerebrospinal fluid and also with lymphocytes.
6- Unlike the description done so far, Teicoplanin micelles have Ptx greater loading capacity than those allowing other type of formulations based on micelles, reaching about 10 mg/ml. However, currently available formulations only reach concentrations between 5 and 6 mg/ml of Paclitaxel.
7- In addition, another advantage is that once the lyophilized formulation of Teico-Ptx is reconstituted, it is able to remain stable, without precipitation of the drug, for at least 35 days.

According to particular embodiments of the invention, the compositions of the invention may be adapted to be administered intravenously (IV) to a patient. Thus, in particular embodiments, the compositions of the invention may be liquid formulations or lyophilized formulations that can be reconstituted with a sterile solution to obtain a sterile, translucent and injectable formulation. While the pharmaceutical compositions of this invention are described specifically for Teicoplanin, Dalbavancin and Oritavancin micelles, those skilled in the art shall conclude, by means of the information of this invention, that other lipoglycopeptide compounds and, in particular, other lipoglycopeptide antibiotics could be used. In particular, according to the information of the invention, other lipoglycopeptide antibiotics having a critical micelle concentration (CMC) of about 80x10⁻⁶M may be used.

According to the results of the inventors of this invention, MW of micelles of Teicoplanin with Paclitaxel ranges from 50 to 70 kDa and Teico-Ptx-Alb complex ranges from about 250 to 300 kDa.

Moreover, the inventors of this invention have found that micelles of Teicoplanin loaded with Dtx of Ptx have greater stability than those not loaded, which shows that a hydrophobic molecule, such as Ptx or Dtx, could regulate by itself the mechanisms of dissociation of the micelle, thereby promoting stabilization of the micellar form. In fact, in dialysis experiments, it was shown a loss of about 25% of molecules of Teicoplanin after 48 hours of dialysis, while for the Teico-Ptx complex is was shown a loss of about 5 to 8%.

Teico-Ptx and Teico-Dtx complexes can interact spontaneously and non-covalently, through a hydrophobic interaction with the albumin molecule, creating a new structure: a highly stable ternary complex. Due to the presence of the albumin molecule, such complexes could trigger a mechanism of transcytosis through endothelial cells into the interstitial medium. Then, similarly to that shown for albumin-Ptx complex (ABI007), the complex may attach to the receptor or to the SPARC proteins located in the area where the tumor tissue is.

Taking into account the above results, the inventors of this invention have developed pharmaceutical compositions, which are a particular object of this invention, which comprise a vehicle made up of molecules of one or more lipoglycopeptide antibiotics forming micelles, selected from Teicoplanin, Dalbavancin and Oritavancin, which allow non-covalent incorporation of cancer drugs with hydrophobic properties and which also may be spontaneously coated with a serum protein, such as albumin. In particular embodiments, albumin may be human albumin, recombinant human albumin, bovine albumin and human albumin having fatty acids. In an especially preferred embodiment, micelles, according to the invention, are coated with human serum albumin, with or without fatty acids, in an antibiotic:albumin ratio of 1:2 or 1:4 (w/w).

In another particular embodiment, this invention refers to a water-soluble pharmaceutical composition in which antibiotic micelles are coated with albumin containing folic acid covalently coupled; even more particularly, the amount of folic acid covalently attached to albumin ranges from 1 to 20% of total albumin.

Likewise, in still more particular embodiments, the lipoglycopeptide-antitumor drug antibiotic complex can be addressed to humans by previous injection in transfusion bags having human albumin, human serum or full human plasma. Thus, in particular embodiments, Teico-Ptx and Teico-Dtx complexes would specifically attach to albumin to produce biologically active ternary complexes Teico-Ptx-Alb and Teico-Dtx-Alb once injected the composition of the invention in transfusion bags having human albumin, human serum or plasma.

The inventors of this invention have found that, due to interaction with Teicoplanin micelles, it is possible to obtain an increase in the solubility of a hydrophobic bioactive agent in an aqueous medium, such as Ptx or Dtx, from less than 1 *µ*g/ml to 10 mg/ml (about 10,000 times). Moreover, since both the complexes forming the Teicoplanin, Dalbavancin and Oritavancin compounds with Ptx or DTX, and those non-covalently attached to albumin, have low CMC (e.g., CMC of Teico-Ptx- Alb is of about 50x10⁻⁶ M), it is achieved that the drug for the treatment of cancer patients increases its life in the bloodstream. This way, it could be increased the therapeutic activity of the composition, since it would have more time to access the tumor area.

Moreover, it is important to highlight that the compositions according to the invention do not require the use of solvents which may produce undesirable effects in cancer patients. For example, the compositions of the invention do not require the addition of Cremophor or Ethanol (50%-50%), as it is the case of the commercial formulation of Taxol™. Both Cremophor and Ethanol are the cause of many undesirable side effects when they are administered to patients.

According to a preferred embodiment, the pharmaceutical composition of this invention is adapted for intravenous (IV) administration. Even more preferably, it is a stable and translucent intravenous composition. In particular, the water-soluble pharmaceutical composition of this invention can be lyophilised or it is already in a lyophilized form. In the latter, the composition may be reconstituted with a solvent, such as distilled water, saline (0.9% NaCl), phosphate buffered saline solution (PBS), distilled water containing 5% of dextrose and saline with dextrose at 5%.

It is also a particular object of this invention to produce a sterile and lyophilized pharmaceutical composition which can be re-suspended in the above solvents, so that anti-tumor drugs, such as Dtx or Ptx can reach a final concentration ranging from 0,1 to 20 mg/ml, preferably from 1 to 15 mg/ml, and even more preferably, from 5 to 10 mg/ml.

According to this invention, the pharmaceutical composition has a substance capable of forming micelles selected from Teicoplanin, Dalbavancin and Oritavancin. In a preferred embodiment of this invention, the substance capable of forming micelles is Teicoplanin. All glycopeptides share a heptapeptide core, which allows them to act as inhibitors of cell wall synthesis by the inactivation of transglucosylation and transpeptidation processes of peptidoglycan chains. Changes on the core cause changes in the activity of different synthetic drugs. Thus, dalbavancin, oritavancin and telavancin are semi-synthetic derivatives of teicoplanin, cloroeremomicina and vancomycin.

Please find hereinbelow a brief description of these three substances.

Teicoplanin is a glycopeptide chemically related to vancomycin, having a wide range of activity against Gram-positive bacteria. Actually, Teicoplanin is a glycopeptide complex because it is made up of a linear heptapeptide containing three molecules of p-OH-phenylglycine, 2 molecules of di-OH-phenylglycine and 2 of tyrosine, linked by means of a glycosidic interaction with a mannose and two molecules of acetylglucosamine, with MW of about 1.564 kDa. When this molecule interacts with fatty acids via glucosamine, it becomes a lipo-glycopeptide. There are 5 compounds, when fatty acids (R) are:
R: 4-decenoic acid with MW of 1877.67
R: 8-methyl-nonanoic acid with MW of 1879.68
R: n-decanoic acid with MW of 1879
R: 8-methyl-decanoic acid with MW of 1893
R: 9-methyl-decanoico acid with PM of 1893

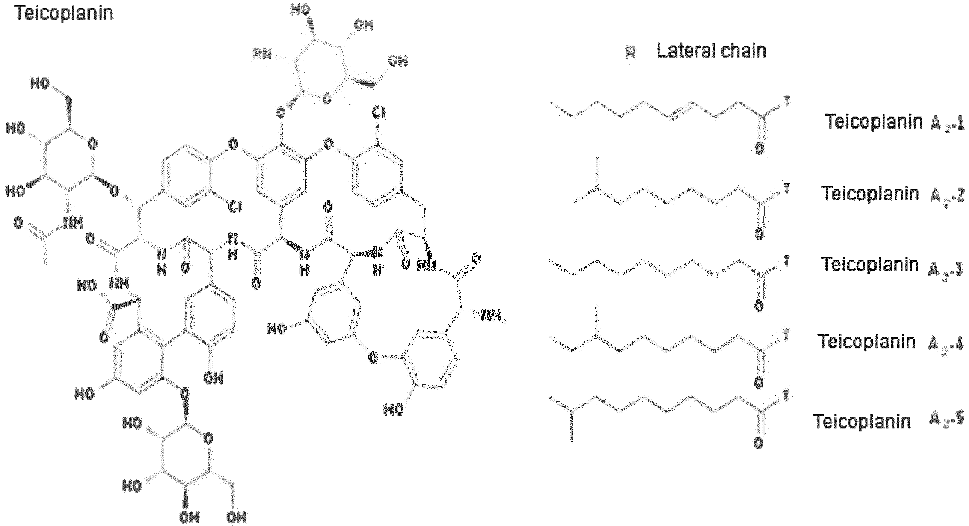

Dalbavancin CAS Registry [171500-79-1] is the generic name of the compound of the following structural formula:

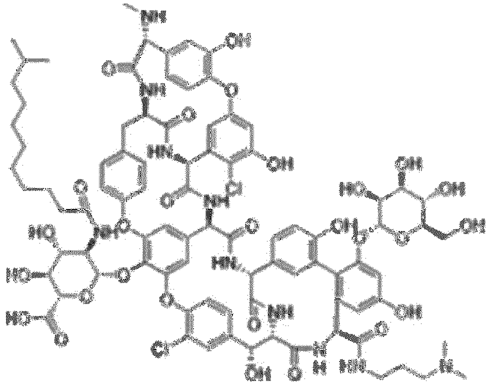

Oritavancin CAS Registry [171099-57-3] is the generic name of the compound of the following structural formula:

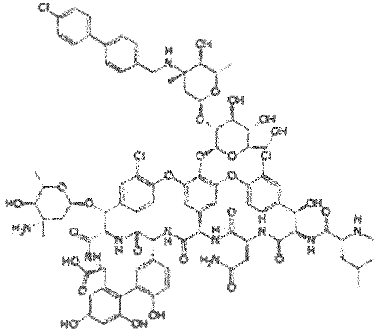

In a particular embodiment, the pH of Teicoplanin micelles ranges from 6 to 8. In a further preferred embodiment, the pH of micelles ranges from 6 to 7.5.

In particular, the water-soluble pharmaceutical compositions of this invention have an averaged size lower than 200 nm; even more particularly, lower than 100 nm, preferably between 5 nm and 80 nm, and even more preferably between 5 nm and 25 nm.

Moreover, the formulations of this invention can be prepared using a passive loading method. For example, bioactive agents can be encapsulated in micelles of antibiotic at higher concentrations than current ones, by means of a simple method, such as incubation at room or cold temperature; during such method, addition takes place spontaneously. Furthermore, the coating of the micelle of antibiotic-Ptx or antibiotic-Dtx with human albumin occurs spontaneously and non-covalently, through a hydrophobic interaction. Eventually, this coating produces a ternary complex made up of antibiotic-Ptx-Alb or antibiotic-Dtx-Alb.

A method used in obtaining the composition of the invention, exemplified for Teicoplanin, can be summarized as follows: The lyophilized Teicoplanin is diluted in distilled water by gentle and slow stirring at a temperature of 4 C for at least 12 hours. Then, Teicoplanin micelles are incubated with a tenth of its volume (1/10 vol/vol) with solvents such as ethanol or dimethylsulfoxide, which have the hydrophobic anti-tumor drugs Ptx or Dtx completely solubilized. The samples are gently stirred and incubated at room temperature for at least 4 hours, until the solution becomes completely clear. Then, the organic solvent is removed from Teicoplanin micelles by dialysis. This dialysis is performed with a pharmaceutical-grade solution with a suitable pH (between pH 6 and pH 7.5). The micelle formulation with the drug added, essentially solvent free and with the desired pH, is centrifuged at 15,000 x g for 10 minutes, in order to remove any hydrophobic insoluble compound that was not incorporated to micelles. Then, the clear aqueous micelle formulation containing encapsulated antifungal or anti-tumor drugs are incubated with purified human albumin, human plasma or human serum, in order to guarantee the interaction of albumin with the loaded micelles. This incubation can be conducted at room temperature for at least 2 hours. Finally, the formulations made up of Teico-Ptx or Teico-Ptx-albumin are lyophilised again.

Therefore, this invention also refers to a method for obtaining micelles containing, at least, an encapsulated hydrophobic bioactive agent. Such method comprises the following steps:
(a) Making soluble the antibiotic in distilled water or in a saline solution at a pH ranging from 5 to 7.5, always above CMC, and then maintaining the solution with gentle stirring at 4 C for at least 12 hours;
(b) The, it is added to the Teicoplanin micelle solution obtained on the previous step a solution of the bioactive agent chosen at 10% of dimethylsulfoxide or ethanol;
(c) Incubating such mixture at room temperature between 2 and 12 hours, in order to guarantee the correct incorporation of the bioactive agent to micelles;
(d) Dialyzing the solution of micelles containing the bioactive agent with a solution of distilled water or a proper pharmaceutically solution having a pH ranging from 5 to 7.5 for 12 hours at 4 C temperature, in order to remove the organic solvent;
(e) Incubating the micelles of the complex Teicoplanin-drug with human albumin for one or two hours at room temperature, in order to allow the formation of the ternary complex Teicoplanin-drugs- albumin;
(f) Sterilizing the aqueous and clear solution obtained in the previous step by filtration at 0.22 micron; and
(g) Lyophilizing the sterile micelles.

For intravenous administration, the lyophilizate obtained by means of the above method may be suspended in a proper pharmaceutically solution.

The amount of different bioactive agents incorporated in Teicoplanin micelles can be determined using proper spectroscopic or chromatographic techniques, such as high-pressure liquid chromatography (HPLC).

According to this invention, micelles can be loaded with anti-tumor agent, e.g. Ptx or Dtx previously solubilized in organic solvents, such as ethanol or dimethylsulfoxide. In particular embodiments, the amount of ethanol or dimethylsulfoxide used to incorporate Dtx and Ptx into Teicoplanin micelles ranges from 5 to 15% of the total volume.

The solvents used to load the micelles with therapeutically active substances can be removed by a method adapted for this purpose. For example, they can be removed by dialysis or by molecular filtration using Sephadex G25.

The person skilled in the art will understand that, by means of the information of this invention, it shall be possible to incorporate into the composition of the invention drugs other than Ptx and Dtx. This way, other drugs may be incorporated into the compositions of the invention; such drugs are those defined by an octanol/water partition coefficient higher than 1, preferably higher than 5. Representative drugs in this category include prostaglandins, testosterone, estradiol, vitamin e, cortisone and dexamethasone. In addition, the compositions of this invention could incorporate hydrophobic antifungal drugs, such as Amphotericin B. Micelles formed by antibiotics, such as lipo-glycopeptide Teicoplanin and other antibiotics such as cyclic lipopeptide Daptomycin, show different capacity to incorporate Dtx or Ptx into them, wherein Teicoplanin shows twice load capacity than Daptomycin. In this sense, the results obtained by the inventors of this invention clearly show that the structures formed by each micelle are clearly different, resulting in a change in the capacity to make soluble Dtx or Ptx.

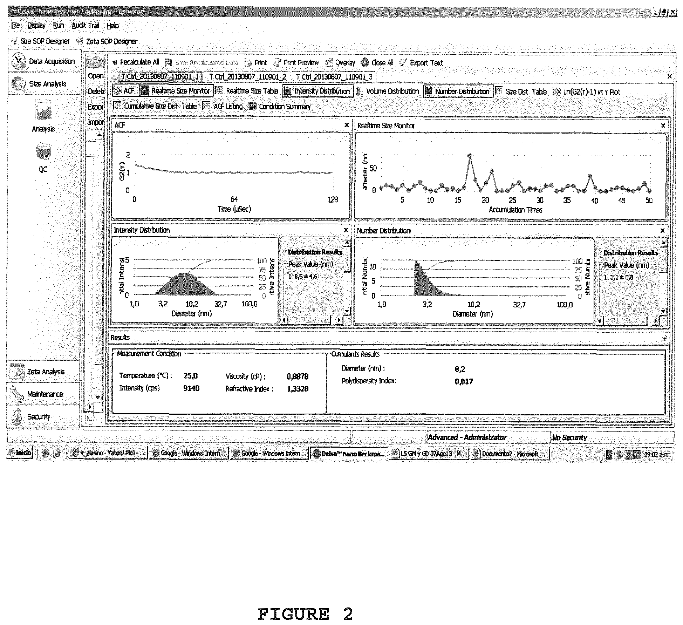

A study by electron microscopy shows that Teicoplanin micelles of this invention are spherical. Moreover, the results show that the formation of the complex Teico-Ptx does not produce any substantial change in the size of the micelle. See, for example, figures 2, 3 and 4.

A comparative study on the properties and features of the micelles of a lipoglycopeptide, Teicoplanin (Teico) and the micelles of an antibiotic made up of a cyclic lipopeptide such as Daptomycin (Dapto) highlights the differences and advantages between them, being differently not only structurally but also taking into account the features as nanocarriers. From the information obtained in books and experimental results by the inventors of this invention, the following aspects can be highlighted.
1- Teicoplanin micelles have a half life ranging between 70 and 100 hours, which is much higher than Daptomycin micelles, which have a half life between 7 and 10 hours.
2- Regarding micelle stability, while Daptomycin micelles are stable and biologically active for 12 hours at 25°C and for 48 hours at 4°C, Teicoplanin micelles are stable for 48 hours at 25°C and for 35 days at 4°C.
3- The suggested maximum doses of Daptomycin to avoid unwanted side effects or cytotoxicity are from 10 to 12 mg/kg/ day. For Teicoplanina, however, the suggested maximum dose is of 20 mg/kg/day, which implies the possibility of applying a higher amount of Teicoplanin to channel higher drug doses.
4- In relation to point 3 above, the results obtained by the inventors of this invention show that the micelles of both molecules, Teicoplanin and Daptomycin, at the same concentration, can introduce Paclitaxel (Ptx) or Docetaxel (Dtx). However, while the maximum load for Teicoplanin is of 10 mg/ml, the maximum load for Daptomycin is only of 5 mg/ml.
5- Regarding the stability over time of Teico-Ptx or Dapto-Ptx complexes, the results obtained by the inventors of this invention show that Dapto-Ptx complex becomes unstable releasing Ptx, which precipitates within 48 hs. On the other hand, Teico-Ptx complex remains stable for, at least, 35 days.
6- Regarding the size of the micelles of both molecules, the results obtained by the inventors of this invention show that Dapto and Dapto-Ptx micelles have a diameter of 5 and 5.5 nm, while those of Teico and Teico -Ptx are 40% larger, since they have an average diameter, measured by the technique "dynamic light scattering", of 8 and 9 nm, respectively.
7- Finally, the critical micelle concentrations for each case show that Teico micelles have CMC of 100 *µ*M, while Dapto micelles at pH 4 have CMC of 400 *µ*g/ml (250 *µ*M). However, when Dapto becomes soluble at pH 6.5, Dapto molecule can remain soluble even at concentrations of 150 mg/ml. This issue is also very important to think about a stable formulation to transport hydrophobic drugs, since the higher CMC, the higher instability of the complex which produces a faster release of the drug by dilution in the bloodstream, as in the case of Daptomycin, which is unfavorable for a good transport system of intravenous drugs.

An essay has recently been published: "Characterization of cell-penetrating lipopeptide micelles by spectroscopic methods". Gehne S, Sydow K, Dathe M and Kumke MU. J PhysChem B. 2013 Nov. 21;117 (46):14215-25, wherein it is described the synthesis of another lipopeptide. The authors mention the formation of micelles of anionic and cationic lipopeptides. They mention studies to assess their interaction with capillary endothelial cells. Within the studies, it is described that cationic derivatives are very strong surfactants with low CMC in the micromolar range (0.5 to 14 uM). The combination of micelle-forming property and the cell penetration capacity could prepare the ground for the development of a new class of drug support efficient systems. In this essay, such lipopeptides have only been assessed regarding their physicochemical properties but they have not been studied as carriers of hydrophobic cancer drugs.

### EXAMPLES

### Example 1

### Paclitaxel loading into teicoplanin micelles

Samples with 400 mg of teicoplanin were dissolved in 2 ml of distilled water at pH 5.5 or in 2 ml of phosphate buffer solution of 20 mM at pH 6, with gentle stirring in order to obtain full dissolution. The solution was allowed to stand for at least 4 hours at 4°C. A 0.5 ml aliquot was incubated with 25 *µ*l of DMSO having increasing amounts of Paclitaxel (Ptx) in order to reach the following final concentrations: 1 mg/ml, 5 mg/ml, 10 mg/ml and 20 mg/ml.

The solutions were incubated at 25°C for at least 4 hours and then centrifuged at 15,000 xg for 10 minutes to remove the potential insoluble Ptx that had not encapsulated in teicoplanin micelles. Finally, in order to remove the remaining DMSO, the samples were dialyzed with a phosphate buffer solution of 20mM at pH 6 for 12 hours. The quantification of Ptx introduced into teicoplanin micelles was carried out by using HPLC.

**Table I: Introduction of Ptx into Teicoplanin micelles**

| Teicoplanin | Ptx concentration | Ptx in Teicoplanin micelles |
|---|---|---|
| 200 mg/ml | 1 mg/ml | 1 mg/ml |
| 200 mg/ml | 2.5 mg/ml | 2.5 mg/ml |
| 200 mg/ml | 5 mg/ml | 5 mg/ml |
| 200 mg/ml | 10 mg/ml | 10 mg/ml |
| 200 mg/ml | 20 mg/ml | 2 mg/ml |

When the concentration of Ptx is increased by 20 mg/ml, the complex becomes unstable, releasing all Ptx, which immediately precipitates.

### Example 2

### Effect of temperature on Ptx loading into Teicoplanin micelles

Ptx is loaded into teicoplanin micelles at a concentration of 5 mg/ml, according to the description of Example 1, but for 30 minutes at 4°C, 25°C and 37°C. After incubation, the samples were centrifuged at 15,000 xg for 10 minutes and they were also dialyzed in distilled water for 12 hours at 4°C. Finally, the amount of Ptx introduced into the teicoplanin micelle in a soluble way was quantified by HPLC. The results show that the change in the temperature at which Ptx is loaded does not produce a significant increase in introduction. The load in all conditions was higher than 90%.

**Table 2: Effect of temperature on the introduction of Ptx into Teicoplanin micelles**

| Temperature | Teicoplanina | Ptx added | Ptx loaded (%) |
|---|---|---|---|
| 4°C | 100 mg/ml | 5 mg | 94 |
| 25°C | 100 mg/ml | 5 mg | 96 |
| 37°C | 100 mg/ml | 5 mg | 98 |

### Example 3

### Chromatographic profile in Sephadex G200 of micelles of Teico-Ptx and of Teico-Ptx complexed with Albumin

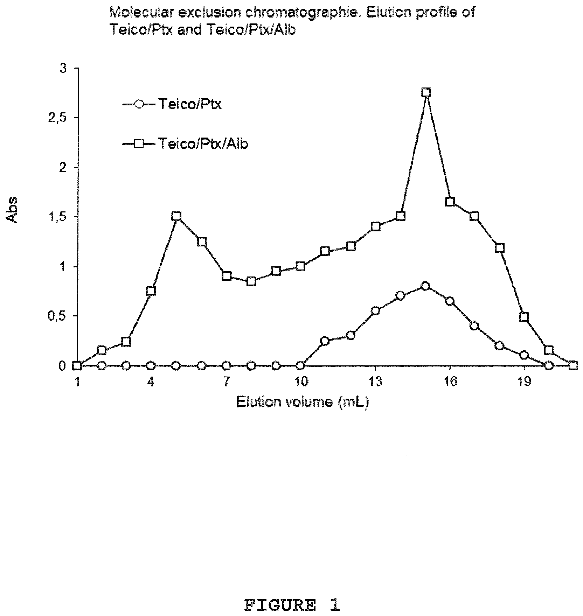

Teico-Ptx micelles having 200 mg/ml of Teico and 5 mg of Ptx were prepared as described in the above examples. A part of these micelles were incubated with a volume of human serum albumin of 200 mg/ml. (Laboratory of Hemoderivatives of the National University of Cordoba) at 37°C for 3 hours. Finally, they were chromatographed in Sephadex G200 to determine the elution profile of each preparation. See Figure 1

A volume of 200 ul of Teico micelles (200 mg/ml) was loaded with Ptx to reach a final concentration of 5 mg/ml of Ptx. Then, 200 ul of a solution of Teico micelles (200 mg/ml) loaded with 5 mg/ml of Ptx was incubated in the presence of 200 ul of 200 mg/ml of albumin for 4 hours to generate the ternary complex Teico-Ptx-Alb. After that, each preparation was chromatographed in Sephadex G200. The cultivated volume was of 250 ul. Vo of the column is of 5 ml determined by using blue dextran. As it can be seen, Teico-Ptx micelles elute with apparent hydrodynamic radius of 50 kDa (black circles). However, when the complex was incubated with albumin to form ternary complexes Teico-Ptx-Alb, it show absorbance at 280 nm to detect albumin (red circles). This result shows that the attachment of albumin to Teico-Ptx micelles produces a change in the MW of 50 kDa, to a MW of about 200 kDa.

### Example 4

### In vitro Comparative study of the cytotoxic activity of Teico-Ptx versus Ptx in tumor cell culture.

Tumor cells, such as HEP-2, were incubated in MEM medium having fetal bovine serum by 2% (Natocor-Villa Carlos Paz - Cordoba - Argentina) in a CO₂ incubator by 5% CO₂, at confluence. Different concentrations of the compositions mentioned below were added to the cell culture:
a) - Ptx (positive control): Ptx Control in DMSO at concentrations of 5, 10, 20 and 50 nM.
b) - Micelles of Teico-Ptx: The Ptx on Teico micelles is at the same concentrations than the ones of Ptx control.
c) - Micelle of Teico-Ptx-Alb and Teico-Ptx, at the same concentrations than in item b), coated with Albumin, with a Teico-Alb ratio of 1/1 (w/w).
d) - Teicoplanin Control Micelles: Teico concentration equal to the ones used in all previous experiments (5 *µ*g/ml).

Cell viability was assessed by MTT test after 24 hours of incubation.

### Cell death after 24 hours of treatment

| Ptx ( ) | Ptx-Control | Teico-Ptx | Teico-PTX-Alb | Teico 5 *µ*M |
|---|---|---|---|---|
| 5 nM | 10% | 15% | 14% | 0% |
| 10 nM | 25% | 35% | 31% | 0% |
| 20 nM | 40% | 55% | 60% | 0% |
| 50 nM | 90% | 95% | 92% | 0% |

The results show that Ptx can be released from teicoplanin micelles, even in the presence of albumin, to kill culture cells. Teicoplanin by itself has no effect on any cell type, while Ptx control in DMSO and on the formulation Teico-Ptx or the Teico-PTX-albumin ternary complex produce complete killing of tumor cells in 24 hours up to a concentration of 50 ng/ml.

### Example 5

### Determining particle size of Teico, Teico-Ptx and Teico-PTX-Alb micelles by dynamic light scattering (DLS)

In Figure 2, teicoplanin control is shown: The results of the analysis by DLS show a particle size of 8 nm with a polydispersity index of 0.017.

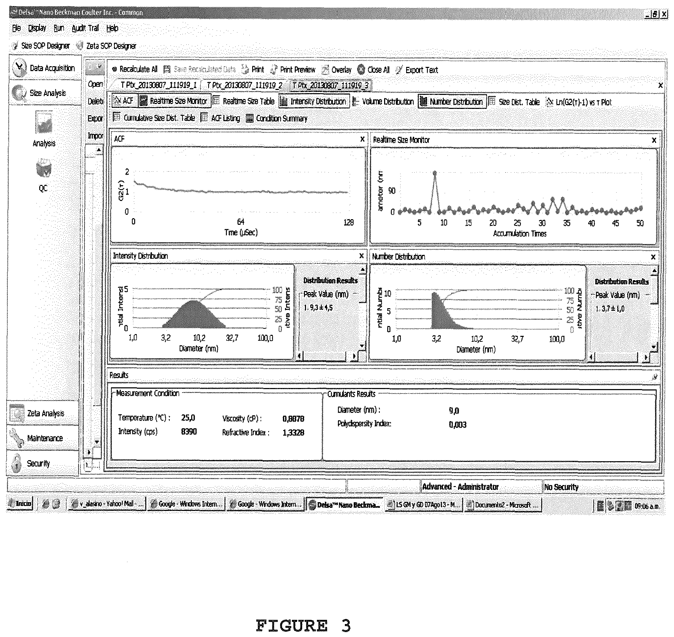

Figure 3 shows the results of the analysis by DLS of Teico-Ptx complex. In this case, the particle size was of 9 nm and the polydispersity index was of 0.003.

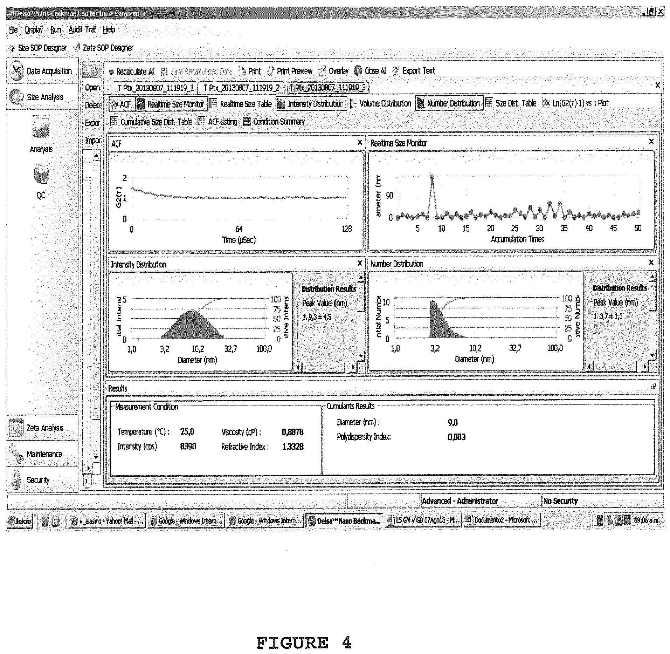

Figure 4 shows the results of the analysis by DSL of Teico-PTX-Alb complex. The particle size was of 14.5 nm and polydispersity of 0.25. This result shows an increase in comparison with Teico and Teico-Ptx complex.

### Example 6

### Electron microscopy of Teico, Teico-Ptx and Teico-Ptx-Alb micelles

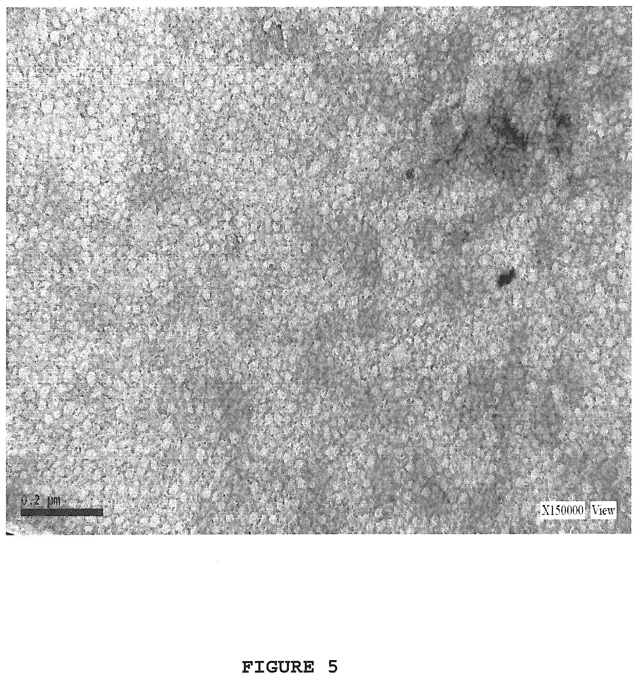

Micelles were prepared according to Examples 1-3 and then they were mounted onto grids for electron microscopy observation. See Figures 5, 6 and 7.

Figure 5 shows an electron photomicrograph at 150,000 x of Teicoplanin micelles.

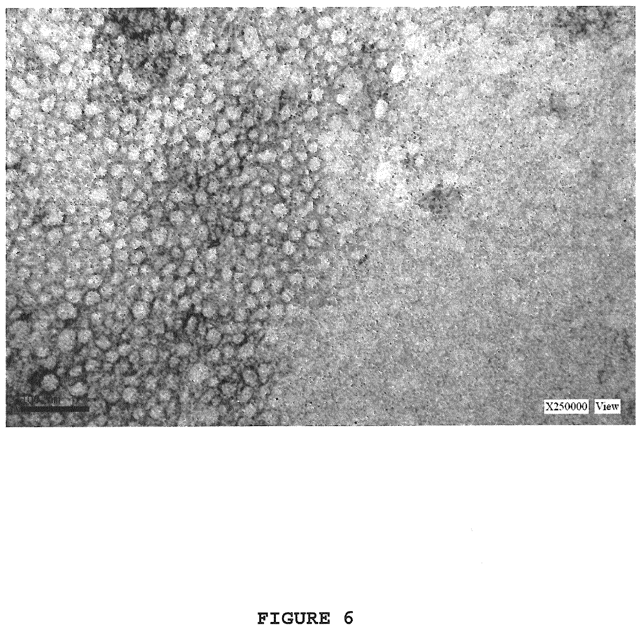

Figure 6 shows an electron photomicrograph at 250,000 x of Teicoplanin-Ptx complex. The black bar represents 100 nm

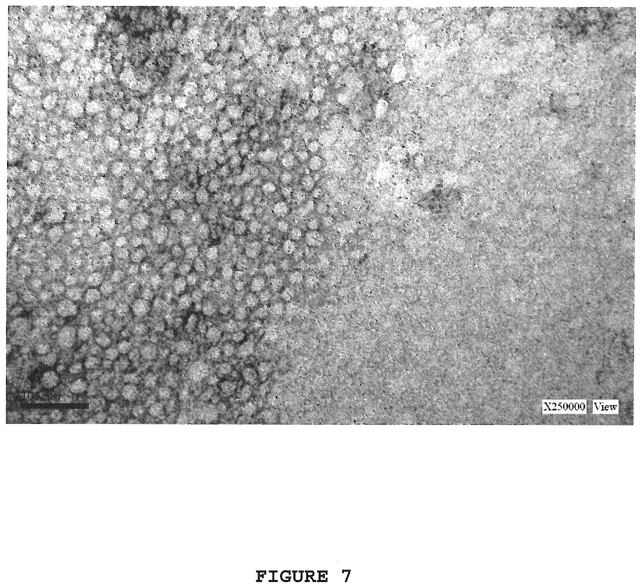

Figure 7 shows an electron photomicrograph at 250,000 x of Teicoplanin-Ptx-Albumin complex. The black bar represents 100 nm

## Claims

1. A water-soluble pharmaceutical composition comprising at least a therapeutically active substance and at least a substance capable of forming micelles, wherein such therapeutically active substance is selected from drugs with hydrophobic characteristics for the treatment of cancer patients and wherein such substance capable of forming micelles is selected from Teicoplanin, Dalbavancin and Oritavancin compounds.

2. A water-soluble pharmaceutical composition according to the above claim, wherein such drugs with hydrophobic characteristics for treating cancer patients are selected from antitumor drugs.

3. A water-soluble pharmaceutical composition according to the above claim comprising at least an antitumor drug with hydrophobic characteristics and at least a substance capable of forming micelles selected from Teicoplanin, Dalbavancin and Oritavancin compounds, wherein such compounds are forming micelles and wherein such antitumor drug with hydrophobic characteristics is attached to the micelles non-covalently.

4. A water-soluble pharmaceutical composition according to claim 2 comprising an antitumor drug with hydrophobic characteristics and a substance capable of forming micelles, wherein such antitumor drug is Paclitaxel and wherein such substance capable of forming micelles is Teicoplanin.

5. A water-soluble pharmaceutical composition according to the above claim comprising Paclitaxel and Teicoplanin according to a molar ratio ranging from 1:5 to 1:100.

6. A water-soluble pharmaceutical composition according to the above claim comprising between about 1 mg/ml and 10 mg/ml of Paclitaxel and 10 mg/ml and 200 mg/ml of Teicoplanin.

7. A water-soluble pharmaceutical composition according to claim 2 comprising an antitumor drug with hydrophobic characteristics and a substance capable of forming micelles, wherein such antitumor drug is Docetaxel and wherein such substance capable of forming micelles is Teicoplanin.

8. A water-soluble pharmaceutical composition according to the above claim comprising Docetaxel and Teicoplanin according to a molar ratio ranging from about 1:5 to 1:100.

9. A water-soluble pharmaceutical composition according to the above claim comprising between about 1 mg/ml and 10 mg/ml of Docetaxel and 10 mg/ml and 200 mg/ml of Teicoplanin.

10. A water-soluble pharmaceutical composition according to any of the above claims that it is a sterile injectable composition.

11. A water-soluble pharmaceutical composition according to any of the above claims that it is a lyophilized composition.

12. A water-soluble pharmaceutical composition according to the above claim, wherein such lyophilized composition can be reconstituted with a solvent selected from a solution of distilled water, a saline solution (NaCl 0.9%) a phosphate saline buffer solution (PBS), distilled water containing 5% of dextrose, a saline solution with 5% dextrose and human albumin solution by 20%, to obtain a sterile translucent composition.

13. A water-soluble pharmaceutical composition according to claims 1 to 10 that is a liquid composition.

14. A water-soluble pharmaceutical composition according to any of the above claims comprising micelles of at least one selected compound such as Teicoplanin, wherein such micelles are loaded with antitumor drugs with hydrophobic characteristics and wherein such micelles are non-covalently coated with albumin.

15. A water-soluble pharmaceutical composition according to the above claim, wherein albumin is selected from human albumin, recombinant human albumin, human albumin containing fatty acids and bovine albumin.

16. A water-soluble pharmaceutical composition according to the above claim, wherein the antibiotic:albumin ratio ranges from about 1:2 to 1:4 (w/w).

17. A water-soluble pharmaceutical composition according to claim 15, wherein albumin comprises folic acid covalently coupled.

18. A water-soluble pharmaceutical composition according to the above claim, wherein the amount of folic acid covalently attached to albumin ranges from about 1 to 20% of total albumin.

19. A water-soluble, lyophilized and sterile pharmaceutical composition according to any of the above claims, wherein once re-suspended in a solvent selected from a solution of distilled water, a saline solution (NaCl 0.9%) a phosphate buffer solution (PBS), distilled water containing 5% of dextrose, saline solution with 5% dextrose and human albumin solution by 20% allows obtaining a final concentration ranging from 0.1 to 10 mg/ml of an antitumor drug selected from Paclitaxel and Docetaxel.

20. A water-soluble, lyophilized and sterile pharmaceutical composition according to the above claim, wherein once re-suspended in a solvent selected from a solution of distilled water, a saline solution (NaCl0.9%), phosphate buffer solution (PBS), distilled water containing 5% of dextrose, saline solution with 5% of dextrose and human albumin solution by 20% allows obtaining a final concentration ranging from 1 to 10 mg/ml of an antitumor drug selected from Paclitaxel and Docetaxel.

21. A water-soluble, lyophilized and sterile pharmaceutical composition according to the above claim, wherein once re-suspended in a solvent selected from a solution of distilled water, a saline solution (NaCl 0.9%), phosphate buffer solution (PBS), distilled water containing 5% of dextrose, saline solution with 5% of dextrose and human albumin solution by 20% allows obtaining a final concentration ranging from 5 to 10 mg/ml of an antitumor drug selected from Paclitaxel and Docetaxel.

22. A water-soluble, lyophilized and sterile pharmaceutical composition according to claim 11, wherein once dissolved, it has pH ranging between 6 and 8.

23. A water-soluble, lyophilized and sterile pharmaceutical composition according to the above claim, wherein once dissolved, it has a pH ranging between 6 and 7.5.

24. A water-soluble, lyophilized and sterile pharmaceutical composition according to any of the above claims having an average size lower than 200nm.

25. A water-soluble, lyophilized and sterile pharmaceutical composition according to the above claim having an average size ranging between 5 nm and 80 nm.

26. A water-soluble, lyophilized and sterile pharmaceutical composition according to the above claim having an average size ranging between about 5 nm and about 25 nm.

27. A water-soluble pharmaceutical composition comprising at least a therapeutically active substance and at least a substance capable of forming micelles, wherein such therapeutically active substance is selected from antifungal drugs with hydrophobic characteristics and wherein such substance capable of forming micelles is selected from Teicoplanin, Dalbavancin and Oritavancin compounds.

28. A water-soluble pharmaceutical composition according to the above claim, wherein such therapeutically active substance selected from antifungal drugs is Amphotericin B and such substance capable of forming micelles is Teicoplanin.
